# EUROPÄISCHE PATENTANMELDUNG

(11) **EP 3 799 114 A1**
(43) Veröffentlichungstag der Anmeldung: **31.03.2021**
(21) Anmeldenummer: 19200201.2
(22) Anmeldetag: 27.09.2019
(51) Int. Cl.: H01L 23/14, A61N 1/375

(54) **THERMOKOMPRESSION-BONDING VON ELEKTRONISCHEN KOMPONENTEN**

(71) Anmelder: Dyconex AG, 8303 Bassersdorf (CH)
(72) Erfinder: Bihler, Eckardt, 8406 Winterthur (CH); Hauer, Marc, 8610 Uster (CH)
(74) Vertreter: Galander, Marcus

(57) **Zusammenfassung**

Die vorliegende Erfindung betrifft ein Verfahren zur Herstellung eines elektronischen Moduls, umfassend die Schritte: Bereitstellen eines ersten Substrats mit mindestens einer ersten elektrischen Kontaktierfläche, einer elektronischen Komponente mit mindestens einer zweiten elektrischen Kontaktierfläche und einer ersten Materiallage aus einem thermoplastischen Material mit mindestens einer Aussparung, welche sich durch die Materiallage erstreckt; Anordnen des ersten Substrats, der elektronischen Komponente und der ersten Materiallage, wobei die erste Materiallage zwischen dem ersten Substrat und der elektronischen Komponente angerordnet ist, und die mindestens eine erste elektrische Kontaktierfläche, die mindestens eine zweite elektrische Kontaktierfläche und die mindestens eine Aussparung zueinander ausgerichtet sind; und Pressschweißen des ersten Substrats, der elektronischen Komponente und der Materiallage, wobei eine Fügestelle zwischen der mindestens einen ersten elektrischen Kontaktierfläche und der mindestens einen zweiten elektrischen Kontaktierfläche gebildet wird, und die Fügestelle von der ersten Materiallage umfasst oder umschlossen wird.

## Beschreibung

Die vorliegende Erfindung betrifft ein Thermokompression-Bonding-Verfahren zum Fügen von elektronischen Komponenten.

Das Thermokompressionsschweißen (thermocompression bonding, oder "TCB", auch als Pressschweißen bekannt) ist ein in der Elektronik bekanntes Verfahren. Üblicherweise wird es zur Verbindung von Silizium-Chips mit Flip-Chip-Bumps (Kontaktierhügel) auf Substraten (Leiterplatten) verwendet.

Dabei werden die Bumps auf der Chipseite in spiegelbildlich angeordnete Kontaktflächen (Pads) eines Substrates (Leiterplatte) gedrückt und mittels Temperatur und Druck verschweißt. Bestehen die Bumps und die Pads aus reinem Gold und sind sie bei der Fügung sehr sauber, kann bereits bei Drücken von einigen Bar und Temperaturen von 260°C die Ausbildung einer intermetallischen Phase an der Grenzfläche zwischen Bump und Pad nachgewiesen werden. Die Verbindung ist stabil gegen Scherung.

Das Thermokompression-Bonding von Metall zu Metall ist jedoch gegenüber dem Löten von Flip Chips mit lötbaren Bumps oder dem Verbindung über anisotrope Kleber noch wenig verbreitet.

Meist wird nach der Verbindung von Chip und Substrate ein Underfiller aus Epoxid in den Zwischenraum zwischen Chip und Substrat eingebracht und ausgehärtet.

Schwachpunkt der bekannten Fügetechniken für die Mikroelektronik bei der Anwendung in Implantaten und Kathetern ist die elektrische Integrität der Aufbauten gegen das Eindringen von Flüssigkeiten. Insbesondere können durch eindringende Flüssigkeit die Isolationseigenschaften der verwendeten Materialien degenerieren. Insbesondere die in der Elektronik meist verwendeten Epoxidharze sind sehr anfällig gegen Feuchtigkeit. Entweder müssen die elektronischen Komponenten durch Metallgehäuse aus Titan (Stand der Technik für Implantate) oder durch eine andere geeignete zusätzliche Ummantelung (Stand der Technik bei Kathetern) geschützt werden.

Ein weiterer Nachteil der bisher bekannten Aufbauten sind die Grenzflächen zwischen den verschiedenen Materialien (Substrat zu Underfiller, Underfiller zu Silizium Chip). Entlang dieser Grenzflächen können Diffusion und Dendriten Wachstum auftreten, und es können Risse entstehen.

Basierend auf diesem Hintergrund ist es daher eine Aufgabe der vorliegenden Erfindung, einfache Mittel und Verfahren zur Herstellung von elektronischen Modulen zur Verfügung zu stellen, die insbesondere besser und zuverlässiger vor Umwelteinflüssen geschützt sind.

Diese Aufgabe wird durch ein Verfahren mit den Merkmalen des Anspruchs 1, ein elektronisches Modul mit den Merkmalen des Anspruchs 10 und ein Medizingerät mit den Merkmalen des Anspruchs 15 gelöst. Geeignete Ausführungsformen sind in den entsprechenden abhängigen Ansprüchen und der folgenden Beschreibung angegeben.

Gemäß Anspruch 1 wird ein Verfahren zur Herstellung eines elektronischen Moduls zur Verfügung gestellt. Das Verfahren umfasst die Schritte:
- Bereitstellen eines ersten Substrats mit mindestens einer ersten elektrischen Kontaktierfläche, einer elektronischen Komponente mit mindestens einer zweiten elektrischen Kontaktierfläche und einer ersten Materiallage aus einem thermoplastischen Material mit mindestens einer Aussparung, welche sich durch die Materiallage erstreckt,
- Anordnen des ersten Substrats, der elektronischen Komponente und der ersten Materiallage, wobei die erste Materiallage zwischen dem ersten Substrat und der elektronischen Komponente angerordnet ist, und die mindestens eine erste elektrische Kontaktierfläche, die mindestens eine zweite elektrische Kontaktierfläche und die mindestens eine Aussparung zueinander ausgerichtet sind, und
- Pressschweißen des ersten Substrats, der elektronischen Komponente und der Materiallage, wobei eine Fügestelle zwischen der mindestens einen ersten elektrischen Kontaktierfläche und der mindestens einen zweiten elektrischen Kontaktierfläche gebildet wird, und die Fügestelle von der ersten Materiallage umfasst oder umschlossen wird.

Der Begriff "Pressschweißen", auch als Thermokompression-Bonding bekannt, wird im Kontext der vorliegende Beschreibung im dem Fachmann verständlichen Sinne verwendet, er bezeichnet insbesondere eine Fügeverfahren, in welchem zweiten zu fügende Elemente unter Druck und Temperatur miteinander verbunden werden.

Insbesondere wird das Pressschweißen unter uniaxialen Druck und Temperatur durchgeführt.

Weiterhin werden das erste Substrat, die erste Materiallage und die elektronischen Komponente so zueinander ausgerichtet, dass die mindestens eine Aussparung der ersten Materiallage genau zwischen der mindestens einen ersten Kontaktierfläche und der mindestens einen zweiten Kontaktierfläche angeordnet ist. Durch diese Anordnung wird gewährleistet, dass die beiden Kontaktierflächen beim Pressschweißen zu einer Fügestelle verbunden werden, die entstehende Fügestelle aber gleichzeitig von der ersten Materiallage eingeschlossen bzw. verkapselt wird.

Vorzugsweise weisen das erste Substrat und/oder die erste Materiallage Ausrichtmarken auf, anhand die Anordnung bzw. das Ausrichten durchgeführt werden kann.

Gemäß einer Ausführungsform des erfindungsgemäßen Verfahrens ist vorgesehen, dass die erste Materiallage aus einem Flüssigkristall-Polymer besteht oder umfasst. Von den Kunststoffen weisen inerte, thermoplastische Materialien die geringste Permeabilität für Wasser und Gase auf. Insbesondere die Gruppe der Flüssigkristallpolymere (LCP - Liquid Crystal Polymer) sticht aus den Polymeren mit der geringsten Wasser bzw.

Gaspermeabilität auf. Durch die Verwendung von LCP kann die Dichtigkeit von mikroelektronischen Aufbauten deutlich verbessert werden. Dadurch können gleichzeitig die kritischen Dimensionen (Abstände zur Umgebung, Materialstärke) reduziert werden. Gleichzeitig kann das in die bestehenden Verfahren integriert werden und stellt damit eine sehr kostengünstige Umsetzung dar. Da LCP ein thermoplastisches Material ist, können die Grenzflächen bei den thermischen Prozessen aufschmelzen und es werden die Grenzflächeneffekte deutlich reduziert.

Der Begriff "Flüssigkristallpolymer" wird im Sinne der vorliegenden Erfindung im dem Fachmann bekannten und geläufigen Sinne verwendet. Ein "Flüssigkristallpolymer" bezeichnet insbesondere ein aromatisches Polymer, welches in der Schmelze oder in Lösung hochgeordnete bzw. kristalline Bereiche aufweist. Nicht-beschränkende Beispiele umfassen aromatische Polyamide wie Aramid (Kevlar) und aromatische Polyester der Hydroxybenzoesäure wie etwa ein Polykondensat aus 4-Hydroxybenzoesäure und 6-Hydroxynaphthalen-2-carbonsäure (Vectran).

Gemäß einer weiteren Ausführungsform des erfindungsgemäßen Verfahrens ist vorgesehen, dass die mindestens eine erste Kontaktierfläche oder die mindestens eine zweite Kontaktierfläche einen Kontaktier- oder Löthügel (Bump) umfasst, welcher insbesondere Gold oder goldbeschichtetes Kupfer umfasst oder daraus besteht.

Gemäß einer weiteren Ausführungsform des erfindungsgemäßen Verfahrens ist vorgesehen, dass die mindestens erste Kontierfläche als Pad ausgebildet ist, wobei das Pad vorzugsweise Kupfer besteht, und einen Kontaktierhügel umfasst, der vorzugsweise aus Gold oder goldbeschichtetem Kupfer besteht. In einer weiteren Ausführungsform ist die mindestens eine zweite Kontaktierfläche als Pad ausgebildet, vorzugsweise aus Gold, und umfasst optional einen Kontaktierhügel, vorzugsweise aus Gold (Gold-Stud-Bump), aus goldbeschichtetem Kupfer (Copper-Pillar-Bump), verzinntem Gold oder verzinntem Kupfer, oder einen Löthügel (Solder Bump), vorzugsweise aus einem Zinnlot. (z.B. Sn99,Ag0.3,Cu0.7 oder eine andere Mischung von Sn, Ag und Cu, oder z.B. Au80Sn20).

Gemäß einer weiteren Ausführungsform des erfindungsgemäßen Verfahrens ist vorgesehen, dass auf der elektronischen Komponente eine zweite Materiallage aus einem thermoplastischen Material angeordnet und vor, während oder nach dem Pressschweißen mit der elektronischen Komponente gefügt wird. Vorzugsweise wird die zweite Materiallage durch Pressschweißen, also unter Druck und Temperatur, oder durch Kleben mit der elektronischen Komponente gefügt bzw. verbunden. In einer Ausführungsform umfasst die zweite Materiallage ein Flüssigkristall-Polymer oder besteht daraus.

Gemäß einer weiteren Ausführungsform des erfindungsgemäßen Verfahrens ist vorgesehen, dass die zweite Materiallage als ein zweites Substrat oder Abdeckfolie ausgebildet ist.

Gemäß einer weiteren Ausführungsform des erfindungsgemäßen Verfahrens ist vorgesehen, dass das erste Substrat als Leiterplatte ausgebildet ist.

Gemäß einer weiteren Ausführungsform des erfindungsgemäßen Verfahrens ist vorgesehen, dass die elektronische Komponente als integrierter Halbleiterbaustein ausgebildet ist, insbesondere ein ASIC, ein Standard-IC oder ein WL-CSP (Wafer-Level-Chip-Scale-Package).

Gemäß einer weiteren Ausführungsform des erfindungsgemäßen Verfahrens ist vorgesehen, dass das erste Substrat aus einem ersten thermoplastischen Polymer gebildet ist, vorzugsweise ein erstes Flüssigkristall-Polymer, und die erste Materiallage aus einem zweiten thermoplastischen Polymer gebildet wird, vorzugsweise ein zweites Flüssigkristall-Polymer. wobei das erste thermoplastische Polymer eine höhere Glasübergangstemperatur oder Schmelztemperatur als das zweite thermoplastische Material aufweist.

Gemäß einer weiteren Ausführungsform des erfindungsgemäßen Verfahrens ist vorgesehen, dass das Pressschweißen bei einer Temperatur oberhalb des Glasübergangstemperatur, insbesondere oberhalb der Schmelztemperatur, des thermoplastischen Materials, insbesondere der ersten Materiallage, des ersten Substrats oder der zweiten Materiallage, durchgeführt wird.

Gemäß einer weiteren Ausführungsform des erfindungsgemäßen Verfahrens ist vorgesehen, dass das Anordnen und Pressschweißen mittels einer Vorrichtung durchgeführt wird. Die Vorrichtung umfasst vorzugsweise eine oder zwei Halteeinrichtungen, die zum Halten der elektronischen Komponente und/oder des ersten Substrats ausgebildet sind. Vorteilhafterweise ist die Haltevorrichtung oder sind die Haltevorrichtungen, weiterhin dazu ausgebildet, Druck auf die elektronische Komponente und/oder das erste Substrat auszuüben. Vorzugsweise umfasst die Haltevorrichtung ein Heizelement, beispielsweise eine Heizplatte, um die benötigte Wärme für das Pressschweißen zur Verfügung zu stellen. Vorzugsweise umfasst die Vorrichtung weiterhin eine Kamera, mit Hilfe welcher das erste Substrat, die elektronische Komponenten und die ersten Materiallage angeordnet bzw. ausgerichtet werden können, beispielsweise über Ausrichtmarken auf dem ersten Substrat, der elektronischen Komponente oder der ersten Materiallage.

Gemäß Anspruch 10 wird ein elektronisches Modul zur Verfügung gestellt. Das elektronische Modul umfasst:
- eine erstes Substrat mit mindestens einer ersten elektrischen Kontaktierfläche, und
- eine elektronischen Komponente mit mindestens einer zweiten elektrischen Kontaktierfläche,
wobei die elektronische Komponente und das erste Substrat über eine Fügestelle mit einander gefügt sind, und die Fügestelle die mindestens eine erste elektrische Kontaktierfläche und die mindestens eine zweite elektrische Kontaktierfläche umfasst, und wobei die Fügestelle durch eine erste Materiallage aus einem thermoplastischen Material umfasst oder umschlossen ist, welche mit der elektronischen Komponente und dem ersten Substrat gefügt ist.

Gemäß einer Ausführungsform des erfindungsgemäßen elektronischen Moduls ist vorgesehen, dass die erste Materiallage ein Flüssigkristall-Polymer umfasst oder daraus besteht.

Gemäß einer weiteren Ausführungsform des erfindungsgemäßen elektronischen Moduls ist vorgesehen, dass die elektronische Komponente von einer zweiten Materiallage aus einem thermoplastischen Material bedeckt oder umschlossen ist. In einer Ausführungsform ist die zweite Materiallage als Abdeckfolie oder als ein zweites Substrat ausgebildet. In einer Ausführungsform umfasst die zweite Materiallage ein Flüssigkristall-Polymer oder besteht daraus.

Gemäß einer weiteren Ausführungsform des erfindungsgemäßen elektronischen Moduls ist vorgesehen, dass die Fügestelle mindestens einen Kontaktierhügel (Bump) und/oder einen Löthügel (Solder Bump) umfasst. In einer Ausführungsform umfasst der Kontaktierhügel Gold (Gold-Stud-Bump), goldbeschichtetes Kupfer (Copper-Pillar-Bump), verzinntes Gold, oder verzinntes Kupfer oder besteht daraus. In einer Ausführungsform umfasst der Löthügel (Solder Bump) ein Zinnlot (z.B. Sn99, Ag0.3, Cu0.7 oder eine andere Mischung von Sn, Ag und Cu, oder z.B. Au80Sn20).

Insbesondere kann über die Höhe des mindestens einen Kontaktierhügel oder Löthügel ein gewünschter Abstand zwischen elektronischer Komponente und erstem Substrat eingestellt werden.

Gemäß einer weiteren Ausführungsform des erfindungsgemäßen Verfahrens ist vorgesehen, dass die mindestens erste Kontierfläche als Pad ausgebildet ist, wobei das Pad vorzugsweise im Wesentlichen aus Kupfer besteht. In einer Ausführungsform ist die mindestens eine zweite Kontaktierfläche als Pad ausgebildet, wobei das Pad vorzugsweise im Wesentlichen aus Gold besteht.

Gemäß einer weiteren Ausführungsform des erfindungsgemäßen elektronischen Moduls ist vorgesehen, dass die elektronische Komponente als integrierter Halbleiterbaustein ausgebildet ist, insbesondere ein ASIC, ein Standard-IC oder ein WL-CSP (Wafer-Level-Chip-Scale-Package).

Gemäß einer weiteren Ausführungsform des erfindungsgemäßen elektronischen Moduls ist vorgesehen, dass das erste Substrat aus einem ersten thermoplastischen Polymer gebildet ist, vorzugsweise ein erstes Flüssigkristall-Polymer, und die erste Materiallage aus einem zweiten thermoplastischen Polymer gebildet wird, vorzugsweise ein zweites Flüssigkristall-Polymer, wobei das erste thermoplastische Polymer eine höhere Glasübergangstemperatur oder Schmelztemperatur als das zweite thermoplastische Material aufweist.

Gemäß Anspruch 15 wird ein Medizingerät, insbesondere ein implantierbares oder in den Körper einbringbares Medizingerät, zur Verfügung gestellt. Das Medizingerät umfasst dabei das erfindungsgemäße elektronische Modul.

Gemäß einer Ausführungsform ist das erfindungsgemäße Medizingerät als implantierbarer Herzschrittmacher, Kardioverter-Defibrillator, Neurostimulator, Diagnosegerät (Biomonitor) oder Katheter ausgebildet.

Weitere Merkmale und Vorteile der Erfindung werden nachfolgend anhand der Figurenbeschreibung von Ausführungsbeispielen erläutert. Es zeigen:
- Fig. 1: einen Querschnitt durch A) zu fügende Bauteile vor dem Verpressen und B) Gefüge nach dem Verpressen;
- Fig. 2A: einen Querschnitt durch zu fügende Bauteile bei der Platzierung mit einem Thermokompression Bonder;
- Fig. 2B: einen Querschnitt durch das Gefüge nach dem Thermokompression Bonding; wobei die Bauteile (Substrat und elektrische Komponente) in die thermoplastische Schablone eingepresst werden, und das Material der Schablone sich zwischen den Bumps verteilt.
- Fig. 3A: einen Querschnitt durch zu fügende Bauteile vor dem Thermokompression Bonding, wobei ein Bauteil (elektronische Komponente) Solder Bumps aufweist, die auf den Kontaktflächen der elektronischen Komponente angeordnet sind;
- Fig. 3B: einen Querschnitt durch das Gefüge nach dem Bonding der elektronischen Komponente mit Solder Bumps; wobei die elektronische Komponente in die Schablone eingepresst wird, das Material der Schablone sich zwischen den Bumps verteilt, und die Solder Bumps mit den Pads auf dem Substrat verlötet werden;

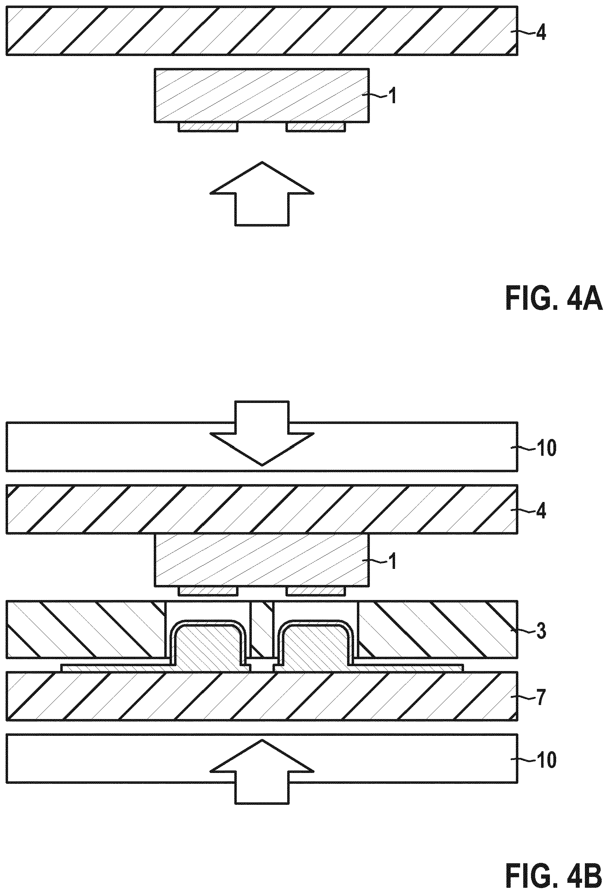

- Fig. 4A: das Aufbringen einer elektronischen Komponente auf ein Substrat;
- Fig. 4B: einen Querschnitt durch die zu fügenden Bauteile vor dem Verpressen mit Druck und Temperatur, und
- Fig. 4C: einen Querschnitt durch das Gefüge nach dem Verpressen mit Druck und Temperatur, wobei die elektrische Komponente komplett in den Substraten eingebettet wird;
- Fig. 5A: einen Querschnitt durch den Stapel aus einer MEMS Komponente mit einer Membran über einer Kavität vor dem Verpressen mit den Heizplatten bzw. Werkzeugen eines Thermokompression Bonders mittels Druck und Temperatur, und
- Fig. 5B: einen Querschnitt durch Gefüge aus MEMS Komponente und dem Substrat nach dem Verpressen, wobei das Material der Schablone an der offenen Seite zu einem Teil herausfließt.

### Beispiele:

Die vorliegende Erfindung betrifft insbesondere ein Thermokompression-Bonding-Verfahren zum Fügen von elektronischen Komponenten 1 mit einer Verkapselung der Fügestellen in Thermoplasten 3.

Durch das erfindungsgemäße Verfahren können vorteilhafterweise elektrische Verbindung von elektronischen, aktiven und passiven Komponenten 1 mit Substraten 7 mittels eines Bondverfahrens gleichzeitig möglichst dichter Verkapselung 3 mit organischen Materialien erzeugt werden.

Vorteilhafterweise kann das erfindungsgemäße Verfahren insbesondere bei der Herstellung von Implantaten ohne Metallgehäuse und Kathetern angewendet werden.

Die erfindungsgemäße Lösung ist dem Stand der Technik hinsichtlich Kosten und Dichtheit bei gleichwertiger Miniaturisierung überlegen und ermöglicht deutlich kostengünstigere Herstellung entsprechender miniaturisierter Implantate und Katheter.

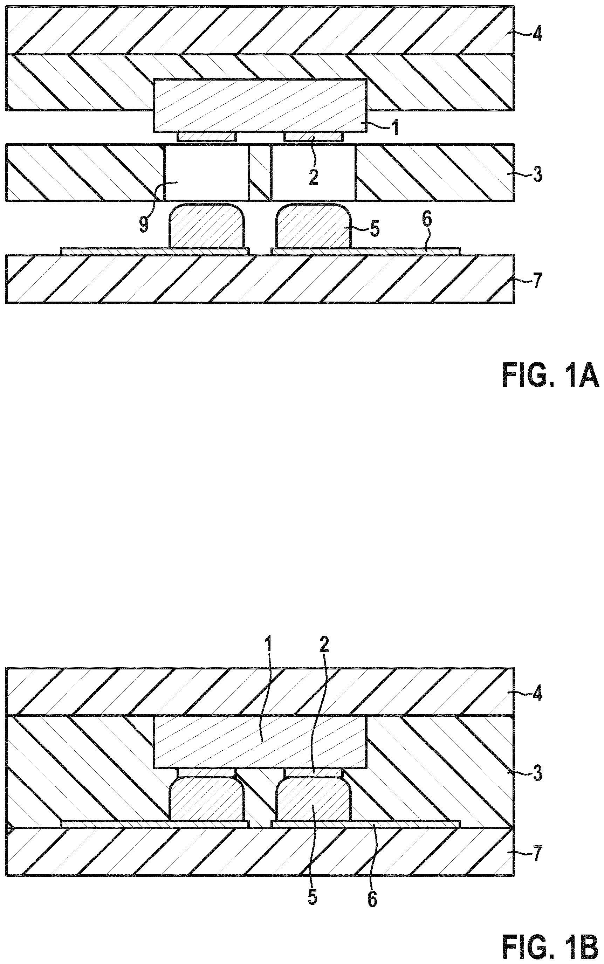

Im Folgenden wird kurz das allgemeine erfindungsgemäße Konzept mit Bezug auf die Figur 1 beschrieben. Auf ein Substrat 7 mit Kontaktpads 6 und darauf gesetzten Gold-Bumps 5 wird eine an den Positionen 9 der Gold-Bumps 5 ausgeschnittene Schablone 3 aus einem thermoplastischen Material (vorzugsweise LCP) gelegt (Figur 1a). Darüber wird passgenau eine elektronische Komponente 1 mit Kontaktpads aus Gold 2 gelegt. Darauf folgt ein Abdeckmaterial 4. Der gesamte Stapel wird unter uniaxialem Druck und Temperatur verschweißt. Nach der Verpressung ergibt sich das in Figur 1b dargestellte Gefüge.

Um eine passgenaue Registration bzw. Ausrichtung der thermoplastischen Schablone 3 zum Substrat 7 und zur elektronischen Komponente 1 zu erreichen, können Löcher in die Schablone 3, das Substrat 7 und die Abdeckfolie 4 so eingebracht worden sein, dass sich alle drei Lagen 3, 4, 7 mittels eines Stiftes präzise aufeinander ausrichten lassen.

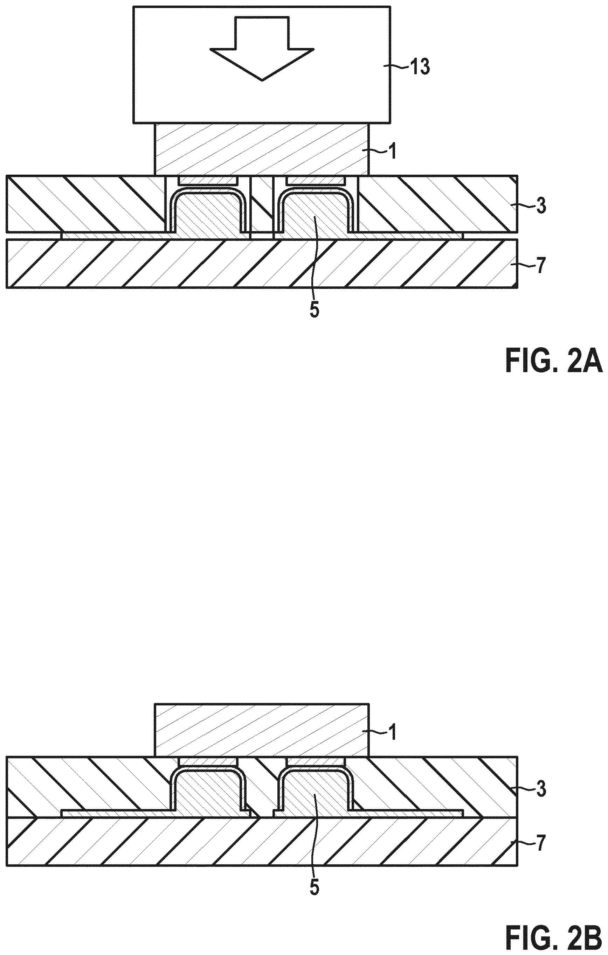

Es ist auch möglich, auf die Abdeckfolie 4 zu verzichten. Die elektronische Komponente 1 wird dann mit einem sogenannten Thermokompression Bonder 13 (Maschinen, die am Markt verfügbar sind) auf die auf dem Substrat 7 ausgerichtete Schablone 3 platziert. Die Registration kann mit einer Kamera auf Ausrichtemarken auf dem Substrat 7 bzw. der Schablone 3 erfolgen. Sobald die Komponente 1 auf der Schablone 3 platziert ist, wird über ein Haltewerkzeug 13 des Thermokompression Bonders Druck ausgeübt und die Temperatur über den Schmelzpunkt der thermoplastischen Schablone 3 gebracht (Fig. 2a).

Das Haltewerkzeug 13 des Thermokompression Bonders hält die elektronische Komponente 1 (z.B. durch im Werkzeug 10 enthaltene Kanäle, die evakuiert werden können) und erlaubt die präzise Positionierung der elektronischen Komponente 1 auf die thermoplastische Schablone 3 und das Substrat 7 über geeignete optische Registrationsmarken in der thermoplastischen Schablone 3 bzw. dem Substrat 7. Herkömmliche verfügbare Thermokompression Bonder können vorteilhafterweise verwendet werden. Vorzugsweise ist das Werkzeug 13 dazu ausgebildet, einen Druck von ca. einigen Bar und ein Temperaturprofil von bis zu 350°C für einige Minuten auszuüben. Das resultierende Gefüge ist in Figur 2b dargestellt.

Das Material der thermoplastischen Schablone 3 ist ein thermoplastisches Polymer, welches durch die Temperatur des Werkzeugs 10 so weich wird, dass er die Zwischenräume zwischen den Bumps 5 und die elektronischen Komponente 1 vollständig verfüllt. Vorzugsweise handelt es sich beim Material der Schablone 3 um das gleiche Material wie auch das Substrat 7. Wenn man LCP (Liquid Crystal Polymer) als Material wählt, ist es möglich das gleiche Material mit einem höheren Schmelzpunkt für das Substrat 7 und mit einem niedrigeren Schmelzpunkt für die thermoplastische Schablone 3 zu verwenden. Temperatur und Druck werden so gewählt, dass einerseits der Schmelzpunkt bzw. Glaspunkt der Schablone 3 überschritten wird, anderseits aber auch eine sichere Verbindung (intermetallische Phase) zwischen den Goldkontaktes der Bumps 5 auf dem Substrat 7 und den Bumps 5 bzw. Chip-Pads 2 auf der elektronischen Komponente 1 erreicht wird. Eine intermetallische Phase zwischen Goldschichten bildet sich bereits bei Drücken von ca. 30 bar und Temperaturen oberhalb von 260°C aus.

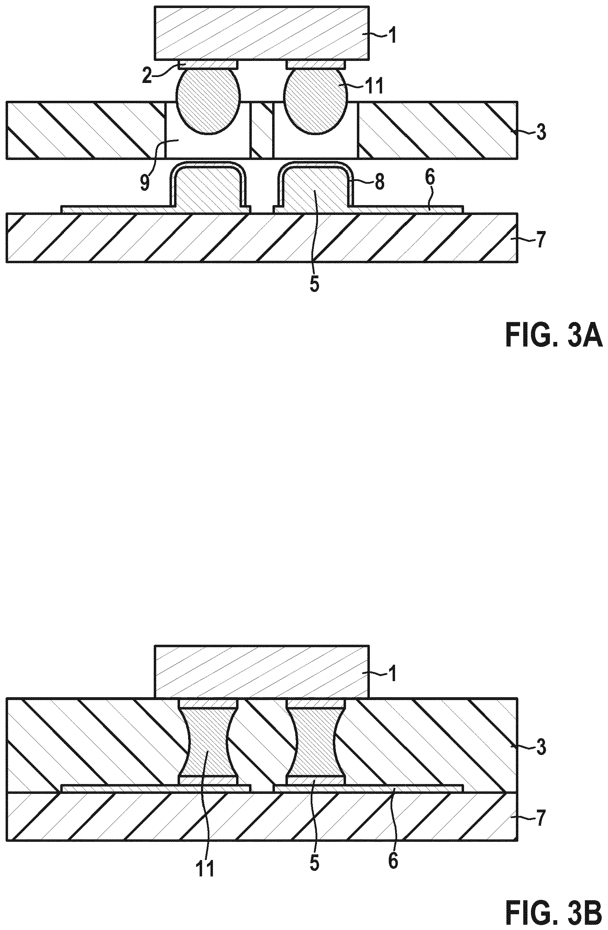

Die elektronische Komponente 1 ist vorzugsweise ein integrierter Halbleiterbaustein (z.B. ein ASIC oder auch ein Standard-IC). Die Metallisierung der IC-Pads 2 ist vorzugsweise mit Gold-Stud-Bumps oder Copper-Pillar-Bumps ausgestattet, alternativ sind auch Solder Bumps 11 möglich. Bei der elektronischen Komponente 1 kann es sich auch um ein WL-CSP (Wafer-Level-Chip-Scale-Package) handeln. Die Bumps 5 können auch aus Kupfer mit einem Überzug aus Gold 8 bestehen (Fig. 3a). Die Höhe der Bumps 5 wird so eingestellt, dass sich beim Bonden der gewünschte Abstand zwischen elektronischer Komponente 1 und Substrat 7 einstellt. Ebenso wird die Dicke der Schablone 3 passend gewählt. Diese Dimensionen werden sich nach den Dimensionen der Pads 2 auf der elektronischen Komponente 1 orientieren.

Wenn die elektronische Komponente 1 über Solder Bumps 11 verfügt, wie das in Fig. 3a dargestellt ist, muss die Temperatur durch das Werkzeug 10 beim Bonding so eingestellt werden, dass sowohl die Schablone 3 fließt als auch die Solder Bumps mit den Bumps 5 auf dem Substrat 7 verlötet werden. Wenn die Solder Bumps 11 genügend groß sind, kann die Höhe der Bumps 5 auf die Höhe der Leiterzüge 6 reduziert werden und nur eine dünne Goldschicht 8 vorgesehen sein. Entsprechend gefügte Bauteile sind in Figur 3b dargestellt.

In einer anderen Ausführungsform wird die elektronische Komponente 1 zunächst auf ein Substrat 4 unter Temperatur und Druck gedrückt und mit dem Substrat 4 verbunden. Alternativ kann die elektronische Komponente 1 auch mit dem Substrat 4 durch einen geeigneten Kleber verbunden werden. Das Substrat 4 besteht vorzugsweise aus einem thermoplastischen Material wie etwa LCP (Liquid Crystal Polymer).

Anschließend wird die so vorbereitete elektronische Komponente 1 auf das Substrat 7 und die thermoplastische Schablone 3 positioniert. Die Positionierung kann durch geeignete mechanische oder optische Registration erfolgen. Der gesamte Aufbau wird zwischen zwei Heizplatten 10 gelegt. Über diese Heizplatten 10 erfolgt eine Verpressung des Stapels mit einem geeigneten Druck- und Temperaturprofil, dessen Verlauf so gewählt ist, dass sowohl die Pads 2 der elektronischen Komponente 1 mit den Bumps 5 auf dem Substrat 7 elektrisch verbunden werden, als auch das thermoplastische Material der Schablone 3 zwischen die Bumps 5 fließt und den gesamten Aufbau verbindet. Die obere Heizplatte 10 kann auch das Werkzeug eines Thermokompression Bonders 13 sein.

Im Unterschied zu den vorangegangenen Ausführungsbeispielen ist hier die elektronische Komponente vollständig in das Material der Substrate 7, 4 und der Schablone 3 eingebettet. Es kann so ein mehrlagiges Substrat bestehend aus den Substratlagen 7 und 4 entstehen.

Auch bei dieser Ausführung können verschiedene Chip-Pad Metallurgien oder Solder Bumps Verwendung finden, wobei lediglich die Prozessparameter für die Verpressung an die entsprechenden Materialien angepasst werden.

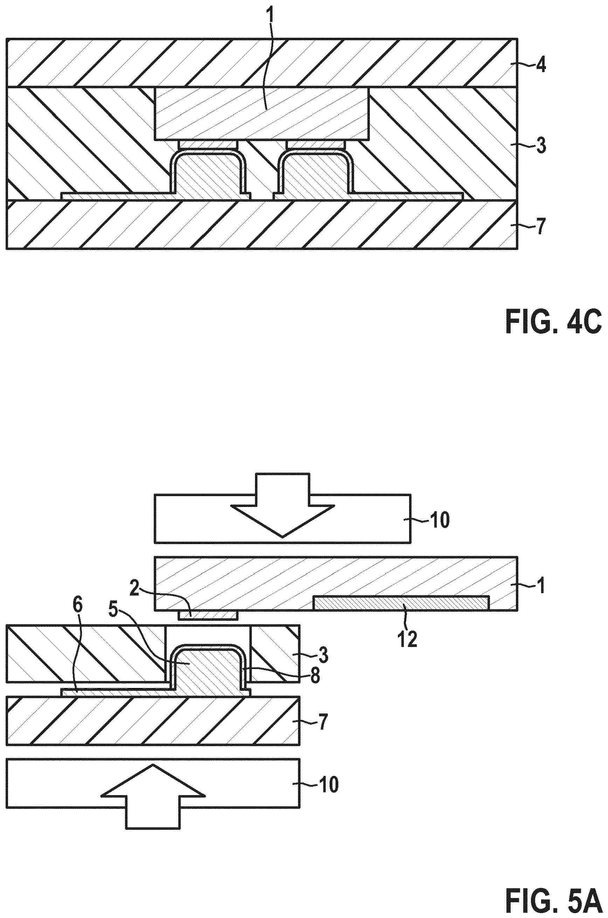

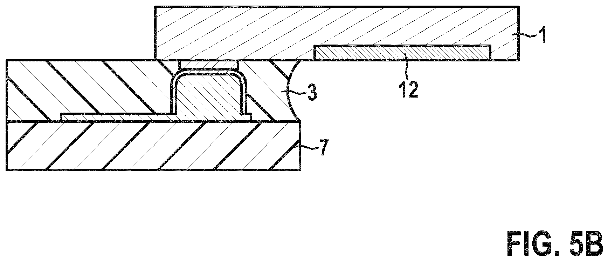

Das erfindungsgemäße Verfahren kann ebenso auf ein MEMS Bauelement (mikromechanisches Bauelement) als zu fügende elektronischen Komponente 1 angewandt werden z.B. mit einer Kavität, die mit einer Membran 12 verschlossen ist, wobei die Membran 12 offen zur Umgebung bleiben soll, Dafür weisen die thermoplastische Schablone 3 und die Substrate 7 bzw. auch 4 vorzugsweise entsprechende Öffnungen auf, damit die Membran 12 nicht abgedeckt wird. Das Werkzeug 13 des Thermokompression Bonders bzw. die Heizplatten 10 werden in diesem Fall so ausgelegt, dass die thermoplastische Schablone 3 nur im Bereich der Bumps 5 für die elektrische Verbindung, nicht jedoch im Bereich der Membran 12 erhitzt wird. Nach dem Verpressen ergibt das in Figur 5b dargestellte Gefüge.

## Patentansprüche

1. Verfahren zur Herstellung eines elektronischen Moduls, umfassend die Schritte:
- Bereitstellen eines ersten Substrats (7) mit mindestens einer ersten elektrischen Kontaktierfläche (6, 5), einer elektronischen Komponente (1) mit mindestens einer zweiten elektrischen Kontaktierfläche (2, 11) und einer ersten Materiallage (3) aus einem thermoplastischen Material mit mindestens einer Aussparung (9), welche sich durch die Materiallage erstreckt,
- Anordnen des ersten Substrats (7), der elektronischen Komponente (1) und der ersten Materiallage (3), wobei die erste Materiallage (3) zwischen dem ersten Substrat (7) und der elektronischen Komponente (1) angerordnet ist, und die mindestens eine erste elektrische Kontaktierfläche (6,5), die mindestens eine zweite elektrische Kontaktierfläche (2, 11) und die mindestens eine Aussparung (9) zueinander ausgerichtet sind, und
- Pressschweißen des ersten Substrats (7), der elektronischen Komponente (1) und der Materiallage (3), wobei eine Fügestelle (6, 5, 2, 11) zwischen der mindestens einen ersten elektrischen Kontaktierfläche (6, 5) und der mindestens einen zweiten elektrischen Kontaktierfläche (2, 11) gebildet wird, und die Fügestelle (6, 5, 2, 11) von der ersten Materiallage (3) umfasst oder umschlossen wird.

2. Verfahren zur Herstellung eines elektronischen Moduls nach Anspruch 1, **dadurch gekennzeichnet, dass** die erste Materiallage (3) aus einem Flüssigkristall-Polymer besteht oder umfasst.

3. Verfahren zur Herstellung eines elektronischen Moduls nach Anspruch 1 oder 2, **dadurch gekennzeichnet, dass** die mindestens eine erste Kontaktierfläche (6) oder die mindestens eine zweite Kontaktierfläche (2) einen Kontaktier- oder Löthügel (Bump) (5, 11) umfasst, welcher insbesondere Gold, goldbeschichtetes Kupfer, verzinntes Gold oder verzinntes Kupfer umfasst oder daraus besteht.

4. Verfahren zur Herstellung eines elektronischen Moduls nach einem der vorherigen Ansprüche, **dadurch gekennzeichnet, dass** auf der elektronischen Komponente (1) eine zweite Materiallage (4) aus einem thermoplastischen Material angeordnet und vor, während oder nach dem Pressschweißen mit der elektronischen Komponente (1) gefügt wird, insbesondere durch Pressschweißen oder Kleben.

5. Verfahren zur Herstellung eines elektronischen Moduls nach Anspruch 4, **dadurch gekennzeichnet, dass** die zweite Materiallage (4) als ein zweites Substrat oder Abdeckfolie ausgebildet ist.

6. Verfahren zur Herstellung eines elektronischen Moduls nach einem der vorherigen Ansprüche, **dadurch gekennzeichnet, dass** das erste Substrat (7) als Leiterplatte ausgebildet ist.

7. Verfahren zur Herstellung eines elektronischen Moduls nach einem der vorherigen Ansprüche, **dadurch gekennzeichnet, dass** die elektronische Komponente (1) als integrierter Halbleiterbaustein ausgebildet ist, insbesondere ein ASIC, ein Standard-IC oder ein WL-CSP.

8. Verfahren zur Herstellung eines elektronischen Moduls nach einem der vorherigen Ansprüche, **dadurch gekennzeichnet, dass** das erste Substrat (7) aus einem ersten thermoplastischen Polymer gebildet ist, vorzugsweise ein ersten Flüssigkristall-Polymer, und die erste Materiallage (3) aus einem zweiten thermoplastischen Polymer gebildet wird, vorzugsweise ein zweites Flüssigkristall-Polymer. wobei das erste thermoplastische Polymer eine höhere Glasübergangstemperatur und/oder Schmelztemperatur als das zweite thermoplastische Material aufweist.

9. Verfahren zur Herstellung eines elektronischen Moduls nach einem der vorherigen Ansprüche, **dadurch gekennzeichnet, dass** das Pressschweißen bei einer Temperatur oberhalb des Glasübergangstemperatur, insbesondere oberhalb der Schmelztemperatur, des thermoplastischen Materials, insbesondere der ersten Materiallage (3), des ersten Substrats (7) oder der zweiten Materiallage (4), durchgeführt wird.

10. Elektronisches Modul umfassend:
- eine erstes Substrat (7) mit mindestens einer ersten elektrischen Kontaktierfläche (6, 5),
- eine elektronischen Komponente (1) mit mindestens einer zweiten elektrischen Kontaktierfläche (2, 11),
wobei die elektronische Komponente (1) und das erste Substrat (7) über eine Fügestelle (6, 5, 2, 11) miteinander gefügt sind, und die Fügestelle (6, 5, 2, 11) die mindestens eine erste elektrische Kontaktierfläche (6, 5) und die mindestens eine zweite elektrische Kontaktierfläche (2, 11) umfasst,
**dadurch gekennzeichnet,**
**dass** die Fügestelle (6, 5, 2, 11) durch eine erste Materiallage (3) aus einem thermoplastischen Material umfasst oder umschlossen ist, und die erste Materiallage (3) mit der elektronischen Komponente (1) und dem ersten Substrat (7) gefügt ist.

11. Elektronisches Modul nach Anspruch 10, **dadurch gekennzeichnet, dass** die erste Materiallage (3) ein Flüssigkristall-Polymer umfasst oder daraus besteht.

12. Elektronisches Modul nach Anspruch 10 oder 11, **dadurch gekennzeichnet, dass** die elektronische Komponente (1) von einer zweiten Materiallage (4) aus einem thermoplastischen Material, insbesondere einer Abdeckfolie oder einem zweiten Substrat, bedeckt oder umschlossen ist, wobei die zweite Materiallage (4) insbesondere ein Flüssigkristall-Polymer umfasst oder daraus besteht.

13. Elektronisches Modul nach einem der Ansprüche 10 bis 12, **dadurch gekennzeichnet, dass** die Fügestelle (6, 5, 2, 11) einen Kontaktierhügel (5) und/oder einen Löthügel (11) umfasst, welcher insbesondere Gold, goldbeschichtetes Kupfer, verzinntes Gold oder verzinntes Kupfer umfasst oder daraus besteht.

14. Elektronisches Modul nach einem der vorherigen Ansprüche 10 bis 13, **dadurch gekennzeichnet, dass** das erste Substrat (7) aus einem ersten thermoplastischen Polymer gebildet ist, vorzugsweise ein erstes Flüssigkristall-Polymer, und die erste Materiallage (3) aus einem zweiten thermoplastischen Polymer gebildet wird, vorzugsweise ein zweites Flüssigkristall-Polymer. wobei das erste thermoplastische Polymer eine höhere Glasübergangstemperatur und/oder Schmelztemperatur als das zweite thermoplastische Material aufweist.

15. Medizingerät, insbesondere implantierbares Medizingerät, umfassend ein elektronisches Modul gemäß einem der Ansprüche 10 bis 14.
